# FASCICULE DE BREVET EUROPEEN

(11) **EP 4 058 097 B1**
(45) Date de publication et mention de la délivrance du brevet: **21.05.2025**
(21) Numéro de dépôt: 20803211.0
(22) Date de dépôt: 12.11.2020
(51) Int. Cl.: A61M 1/36, A61M 1/02, B01D 39/16, B01D 39/18, D04H 1/485, D04H 1/54, D04H 1/72

(54) **UNITE DE FILTRATION POUR LA DELEUCOCYTATION DU SANG COMPRENANT UN NON-TISSE PERFORE EN RELIEF**
FILTRATIONSEINHEIT FÜR DIE LEUKOZYTAPHERESE DES BLUTES MIT EINEM RELIEF-PERFORIERTEN VLIESSTOFF
FILTRATION UNIT FOR THE LEUCOCYTAPHERESIS OF THE BLOOD, COMPRISING A RELIEF-PERFORATED NONWOVEN

(30) Priorité: 15.11.2019 FR 1912763
(43) Date de publication de la demande: 21.09.2022
(73) Titulaire: Maco Pharma, 59420 Mouvaux (FR)
(72) Inventeur: CODDEVILLE, Maxime, 59800 Lille (FR)
(74) Mandataire: Sayettat, Julien Christian
(86) Numéro de dépôt international: PCT/EP2020/081961
(87) Numéro de publication internationale: WO 2021/094483

(56) Documents cités:
- EP-A1- 1 336 417
- EP-A1- 2 671 600
- WO-A1-2004/022831
- GB-A- 2 208 666

## Description

L'invention concerne une unité de filtration destinée à éliminer les leucocytes du sang ou d'un composant sanguin, un procédé de fabrication de ladite unité de filtration comprenant une couche de non-tissé perforé en relief, un procédé extracorporel de déleucocytation à l'aide d'une telle unité ainsi qu'un système à poches comprenant une telle unité.

L'invention s'applique typiquement à la filtration du sang ou d'un composant sanguin, et plus particulièrement à l'élimination des leucocytes d'un sang total ou d'un concentré de globules rouges.

Le sang ou un composant sanguin, après son recueil et sa séparation dans le cas d'un composant, est notamment destiné à être transfusé à un patient qui en a besoin. Lors de cette transfusion, il est bien connu que les leucocytes sont indésirables en ce qu'ils sont susceptibles de provoquer chez le patient des réactions gênantes et/ou potentiellement dangereuses. En effet, les leucocytes augmentent le risque de rejet immunitaire tel que la maladie du greffon contre l'hôte et favorisent la transmission d'agents infectieux.

C'est pourquoi il est recommandé, voire imposé dans certains pays, de déleucocyter le sang ou le composant sanguin préalablement à sa transfusion, et ce dans un rendement donné.

A ce jour, la solution optimale pour éliminer les leucocytes est de filtrer le sang ou le composant sanguin au travers d'une unité de filtration pourvue d'un milieu de déleucocytation.

Un tel milieu de déleucocytation comprend une ou plusieurs couche(s) réalisée(s) en un matériau polymère et choisie(s) de façon à améliorer le taux de déleucocytation, la récupération des composants sanguins, le temps de filtration et/ou la sélectivité de la filtration.

La plupart des milieux de déleucocytation comprend des couches de non-tissés. Un non-tissé est défini comme une feuille manufacturée, constituée de voile ou de nappe de fibres orientées directionnellement ou au hasard, liées par friction et/ou cohésion, et/ou adhésion, à l'exclusion du papier et des produits obtenus par tissages, tricotages, tuftages, couturages incorporant des fils ou filaments de liage ou feutrés par foulage humide, qu'ils soient ou non aiguilletés.

Ces non-tissés sont réalisés par voie fondue ou par filage direct. Le non-tissé filé lié (spun-bond) permet d'obtenir des fibres ayant un diamètre généralement inférieur à 20 µm. Dans le cas de fibres soufflées à l'état fondu (melt-blown) obtenues par une technique d'extrusion soufflage, les fibres ont généralement un diamètre inférieur à 5 µm. Les non-tissés de type spun-bond ou melt-blown possèdent donc une structure relativement dense apte à retenir les leucocytes par un mécanisme d'adsorption et/ou de filtration en surface (ou tamisage).

En plus d'un milieu de déleucocytation, les unités de filtration comprennent également généralement un préfiltre destiné à éliminer les micro-agrégats. Par exemple, dans le document EP 1 336 417, une couche de polyester ayant une perméabilité comprise entre 1000 et 5000 L/m²/s et une taille de pores d'environ 35 µm est disposée en amont du milieu de déleucocytation.

Selon les pays et les habitudes des banques de sang, les composants sanguins peuvent être stockés avant filtration, jusqu'à 14 jours, à 4°C ou à température ambiante, avec ou sans solution additive comme par exemple la solution saline adénine glucose mannitol (SAGM).

Dans ces conditions, les temps de filtration avec des unités de filtration du type de celles décrites dans le document EP 1 336 417 peuvent augmenter jusqu'à une durée pouvant dépasser une heure. Il arrive également que ces unités de filtration colmatent, de sorte que la filtration s'arrête et le composant sanguin est perdu.

Ces colmatages et temps de filtration allongés sont dus essentiellement à la présence d'agrégats formés notamment de globules rouges, de plaquettes, de gel de fibrine. La taille de ces agrégats augmente avec la durée de stockage, pouvant aller de 20 µm jusqu'à 200 µm.

Afin d'éliminer ces agrégats et retenir les leucocytes, le document US 4,923,620 propose une unité de filtration comprenant trois éléments : un voile aiguilleté avec un diamètre de fibre compris entre 20 et 30 µm pour éliminer les gels et agrégats, deux couches intermédiaires ou plus de fibres non-tissées de type melt-blown pour éliminer les micro-agrégats, et plusieurs couches finales de fibres non-tissées de type melt-blown pour éliminer les leucocytes, ces fibres ayant un diamètre plus petit que celui des fibres des couches intermédiaires. Le voile aiguilleté comprend un liant de type acrylique et est compressé à chaud afin de réduire sa taille de pores jusqu'à environ 50 µm.

Le document EP 2 286 821 décrit un matériau de filtre pour éliminer les agrégats comprenant d'une part des fibres courtes ayant un titrage compris entre 0,7 et 4 décitex (dtex) et une longueur comprise entre 1 et 80 mm et d'autre part un tissu de fond comprenant des fibres longues de type spun-bond. Les fibres courtes ont une structure tridimensionnelle et sont enchevêtrées, notamment par jet d'eau, dans les fibres longues, de sorte à obtenir une masse surfacique comprise entre 10 et 80 g/m².

Dans le document WO2013/110694, il est proposé d'utiliser des fibres présentant une cannelure, notamment des fibres trilobées issues de la technologie spun-bond ou melt-blown, afin d'éliminer des produits sanguins les agrégats, les gels, les débris cellulaires et autres fragments.

Enfin, le document WO2015/197955 divulgue un matériau de préfiltre pour filtre à déleucocyter le sang comprenant un voile cardé thermolié constitué d'au moins deux types de fibres discontinues. Ce voile possède une structure suffisamment perméable pour retenir les agrégats sans risque de colmatage tout en ayant une tenue mécanique suffisante pour être intégrée dans une unité de filtration.

En outre, dans le domaine des produits d'hygiène, notamment féminine, il est connu d'utiliser un matériau non-tissé perforé, comme celui décrit dans le document GB 2 208 666, permettant d'améliorer le transfert des liquides vers des matériaux absorbants placés en aval du non-tissé perforé.

La demanderesse a identifié que ce type de matériau non-tissé perforé pouvait être utilisé avantageusement dans des unités de filtration du sang afin de retenir les agrégats et autres gels du sang tout en réduisant de façon significative les risques de colmatage.

A cet effet et selon un premier aspect, l'invention propose une unité de filtration destinée à permettre la déleucocytation du sang ou d'un composant sanguin, comprenant une enveloppe extérieure munie d'au moins un orifice d'entrée et d'au moins un orifice de sortie, l'enveloppe renfermant un élément poreux interposé entre lesdits orifices, ledit élément poreux comprenant au moins un milieu de déleucocytation par adsorption et/ou par filtration des leucocytes et au moins une couche de non-tissé pourvu d'une pluralité de bosses, chacune desdites bosses étant traversées dans la hauteur par une perforation, de sorte à former une couche de non-tissé perforé en relief.

Selon un autre aspect, l'invention porte sur un procédé de fabrication d'une unité de filtration selon le premier aspect de l'invention comprenant
- la formation d'une couche de non tissé perforé par embossage et perçage d'une couche de non-tissé entre deux rouleaux dont l'un au moins est pourvu d'une pluralité d'aiguilles, de sorte à perforer le non-tissé en créant des bosses autour des perforations qui sont en élévation par rapport à la surface du non-tissé,
- l'assemblage de ladite couche de non-tissé perforé en relief avec un milieu de déleucocytation de sorte à former un élément poreux, et
- la disposition dudit élément poreux dans une enveloppe extérieure d'une unité de filtration

L'invention concerne également un procédé extracorporel de déleucocytation du sang ou d'un composant sanguin comprenant le passage dudit sang ou dudit composant du sang au travers d'une unité de filtration selon le premier aspect de l'invention, ledit sang ou ledit composant sanguin étant contenu dans une poche.

Selon un autre aspect encore, l'invention porte sur un système à poches pour la déleucocytation du sang ou d'un composant sanguin comprenant une poche de recueil du filtrat reliée, par l'intermédiaire d'une tubulure et au niveau d'un orifice d'entrée, à un orifice de sortie d'une unité de filtration selon le premier aspect.

D'autres objets et avantages apparaîtront au cours de la description qui suit.
[Fig.1] représente une vue schématique d'une unité de filtration selon l'invention.
[Fig. 2] représente une photographie au microscope électronique à balayage d'une couche de non-tissé filé lié perforé en relief.
[Fig. 3] représente une photographie au microscope électronique à balayage d'une couche de non-tissé de fibres soufflées à l'état fondu, perforé en relief.
[Fig. 4] représente une vue schématique en coupe et de profil d'une tranche de non-tissé perforé en relief.
[Fig. 5] représente une vue schématique d'un système à poches comprenant une unité de filtration selon l'invention.
[Fig. 6] représente la distribution de la taille des pores d'un non-tissé avant perforation.
[Fig. 7] représente la distribution de la taille des pores du non-tissé de la Fig. 6, après perforation.
[Fig. 8] représente la mesure optique d'une bosse d'un non-tissé perforé en relief.

L'unité de filtration selon l'invention est destinée à permettre la déleucocytation du sang ou d'un composant sanguin.

Par composant sanguin, on entend notamment les concentrés de globules rouges, les concentrés plaquettaires, le plasma, le plasma pauvre ou riche en plaquettes, et la couche leuco-plaquettaire ou buffy coat. Les concentrés de globules rouges sont généralement obtenus par centrifugation douce d'une unité de sang total puis extraction de la couche de plasma riche en plaquettes. Dans une variante, les concentrés de globules rouges sont obtenus par centrifugation dure d'une unité de sang total puis extraction de la couche de plasma pauvre en plaquette. Les concentrés de globules rouges sont alors dits "non appauvris en leucocytes et en plaquettes". Dans une autre variante, les concentrés de globules rouges sont obtenus par centrifugation dure d'une unité de sang total puis extraction de la couche de buffy coat et de la couche de plasma pauvre en plaquettes. Les concentrés de globules rouges sont alors dits "appauvris en leucocytes et en plaquettes".

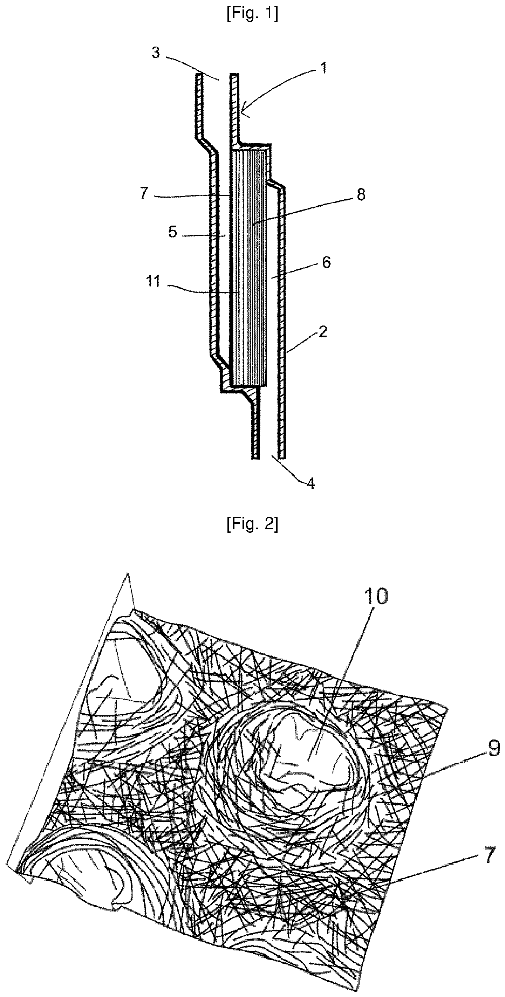

En relation avec la figure 1, l'unité de filtration 1 comprend une enveloppe extérieure 2 munie d'au moins un orifice d'entrée 3 et d'au moins un orifice de sortie 4, l'enveloppe 2 renfermant un élément poreux interposé entre lesdits orifices 3,4. L'élément poreux forme avec l'enveloppe 2 un compartiment d'entrée 5 destiné à recevoir le fluide à filtrer, ledit compartiment d'entrée 5 étant en communication avec l'orifice d'entrée 3, et un compartiment de sortie 6 destiné à recueillir le filtrat, ledit compartiment de sortie 6 étant en communication avec l'orifice de sortie 4.

L'enveloppe extérieure 2 de l'unité de filtration est souple, rigide ou semi-rigide. Par exemple, l'enveloppe est réalisée en polycarbonate, en polychlorure de vinyle ou en une polyoléfine, telle que le polypropylène, le polyéthylène ou un complexe à base de polypropylène.

Le sens d'écoulement du fluide dans l'unité de filtration, de l'entrée 3 vers la sortie 4, permet de définir les termes "amont" et "aval" utilisés dans la description.

L'élément poreux renferme au moins un milieu de déleucocytation 8 par adsorption et/ou par filtration des leucocytes.

Selon l'invention, l'élément poreux renferme en outre au moins une couche de non-tissé 7 pourvu d'une pluralité de bosses 9, chacune desdites bosses étant traversées dans la hauteur par une perforation 10, de sorte à former une couche de non-tissé perforé en relief. Une telle couche est illustrée sur les figures 2 à 4.

Le non-tissé est constitué de fibres enchevêtrées entre elles, mais qui ne sont ni tissées ni tricotées. Le non-tissé est par exemple un non-tissé de fibres soufflées à l'état fondu ou un non-tissé filé lié.

Le non-tissé perforé en relief comprend des fibres thermoplastiques d'un polymère biocompatible tel que le polyester, le polypropylène, le polyéthylène, le polyamide, la cellulose, ou les mélanges de ces polymères. En particulier, les fibres sont des fibres de polyester, notamment de polyéthylène téréphtalate.

Les bosses perforées sont produites par embossage et perçage du non-tissé de part en part. Par exemple, la couche de non-tissé perforé en relief est fabriquée par embossage et perçage d'une couche de non-tissé entre deux rouleaux dont l'un au moins est pourvu d'une pluralité d'aiguilles. De cette façon, le non-tissé est perforé en créant des bosses 9 autour des perforations 10 qui sont en élévation par rapport à la surface du non-tissé. Ces bosses 9 sont creuses et délimitent les perforations. En particulier, les bosses 9 présentent substantiellement toutes les mêmes dimensions et sont formées sur le même côté du non-tissé.

Avantageusement, le non-tissé perforé en relief est un non-tissé filé lié qui présente des propriétés mécaniques avantageuses, notamment en termes de résistance à la traction. Avec ce type de matériau non-tissé filé lié, on peut former par embossage des bosses 9 plus élevées et plus stables qu'avec un non-tissé de fibres soufflées à l'état fondu.

En particulier, les bosses 9 sont de forme tronconique. Les bosses 9 s'élèvent en se rétrécissant à partir d'une surface du non-tissé. D'autres formes tridimensionnelles sont envisageables.

La forme des perforations correspond substantiellement à la forme des bosses. Par exemple, dans le cas d'une bosse tronconique, la perforation est tronconique et les ouvertures proximale et distale de la perforation sont substantiellement circulaires ou elliptiques. Les termes « proximal » et « distal » s'entendent par rapport à la surface du non-tissé.

Les bosses 9 sont séparées les unes des autres. Elles sont réparties sur le non-tissé de façon régulière ou irrégulière. En particulier, les bosses 9 forment sur le non-tissé un motif distinct et répété.

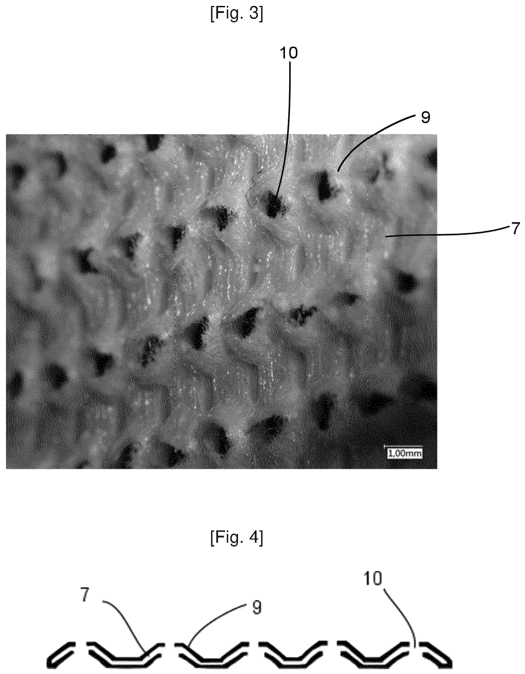

Les figures 2 et 3 montrent une image prise au microscope électronique à balayage un non-tissé perforé en relief, de type filé lié et de fibres soufflées à l'état fondu, respectivement.

De façon inattendue, cette couche de non-tissé perforé en relief possède une bonne capacité à retenir les agrégats et autres gels présents dans le sang ou dans le composant sanguin à filtrer, tout en évitant son colmatage. En outre, on constate que les temps de filtration sont réduits. Les agrégats se forment lors de la conservation du sang et sont composés essentiellement de globules rouges, de plaquettes, de gel de fibrine.

En effet, comme illustré schématiquement sur la figure 4, la présence des bosses 9 perforées en relief dans le non-tissé 7 permet d'obtenir une structure plus épaisse que le même non-tissé non perforé, composée de monts et de vallées dans lesquelles les agrégats peuvent se loger sans bloquer toute la surface de filtration du non-tissé.

Avantageusement, la couche de non-tissé perforé en relief est disposée dans l'unité de filtration en amont du milieu de déleucocytation afin de retenir les agrégats du sang ou du composant sanguin avant la déleucocytation.

Plus particulièrement, plusieurs couches de non-tissé perforé en relief, par exemple deux ou trois couches sont disposées en amont du milieu de déleucocytation.

Encore plus particulièrement, la ou les couches de non-tissé perforé en relief sont disposées dans la partie la plus amont de l'élément poreux de l'unité de filtration.

Le non-tissé perforé en relief est asymétrique. Dans l'unité de filtration, la couche de non-tissé perforé en relief est disposée avec les bosses 9 dirigées vers l'amont ou vers l'aval de l'unité de filtration. Dans le cas où l'unité de filtration comporte plusieurs couches de non-tissé perforé en relief, les couches de non-tissé perforé en relief sont disposées avec les bosses 9 dirigées dans le même sens ou non.

Lorsque les bosses 9 sont dirigées vers l'aval, le flux de sang ou du composant sanguin dans l'unité de filtration est facilité tout en évitant le reflux.

Selon une réalisation, l'épaisseur de la couche de non-tissé perforé en relief est comprise entre 400 et 1 500 µm, mesurée à l'aide d'un micromètre avec une pression de 10 kPa (norme ISO 9073-2:1995). Une épaisseur comprise dans la plage allant de 500 à 1 300 µm est avantageuse pour obtenir une épaisseur suffisante pour piéger les agrégats.

Le non-tissé perforé en relief présente une masse surfacique comprise dans la plage allant de 40 à 90 g/m², notamment compris dans la plage allant de 50 à 80 g/m².

En dessous de 50 g/m², il apparaît que le taux de déleucocytation se dégrade considérablement, du fait de la densité trop faible de fibres. Au-dessus de 90 g/m², la densité de fibre est trop grande et le risque de blocage augmente.

La perméabilité à l'air de la couche de non-tissé perforé en relief est comprise dans la plage allant de 800 à 3 000 L/m²/s.

La perméabilité à l'air est déterminée selon la norme NF EN ISO 9237 sur un échantillon d'au moins 100 cm² à l'aide d'un perméabilimètre à l'air tel que le FX 3300 de TextTest avec une pression d'air réglée sur 196 Pa (Norme EDANA 140.1).

Une perméabilité à l'air supérieure à 3 000 L/m²/s entraîne un temps de filtration plus court, puisque le sang a potentiellement plus de place pour circuler, mais un risque d'augmentation du nombre de leucocytes résiduels. En dessous de 800 L/m²/s, le temps de filtration augmente ainsi que le risque de blocage.

Une perméabilité à l'air de la couche de non-tissé perforé en relief comprise dans la plage allant de 1 300 à 2 500 L/m²/s est un bon compromis entre le temps de filtration, l'occurrence des blocages et le taux de déleucocytation.

La perméabilité à l'air de la couche de non-tissé dépend de plusieurs paramètres dont de la perméabilité à l'air du non-tissé non-perforé de base, des dimensions des perforations et du nombre de perforations dans le non-tissé.

Dans le cas d'un non-tissé de type filé lié, le diamètre moyen des fibres est compris dans la plage allant de 5 µm à 30 µm, particulièrement de 8 µm à 20 µm.

Les dimensions des bosses 9 et des perforations sont déterminées par mesure optique et porométrie.

Les bosses 9 s'étendent à partir de la surface du non-tissé sur une hauteur comprise entre 200 µm et 1 500 µm, en particulier entre 400 µm et 700 µm.

Selon une réalisation, les bosses 9 présentent une ouverture distale, de forme substantiellement cylindrique de diamètre compris entre 100 µm et 800 µm mm, en particulier entre 100 µm et 500 µm. L'ouverture proximale des bosses 9 est de de forme substantiellement cylindrique, de diamètre compris entre 1 000 µm et 3 000 µm, en particulier entre 1 500 µm et 2 500 µm.

Particulièrement, le non-tissé perforé en relief comprend un taux de perforation compris dans la plage allant de 5 à 20 perforations par cm².

Outre la ou les couches de non-tissé perforé en relief, l'élément poreux de l'unité de filtration comprend un milieu de déleucocytation 8 par adsorption et/ou par filtration des leucocytes. Ce milieu de déleucocytation 8 comprend en particulier une ou plusieurs couches 11 d'un matériau non-tissé de fibres soufflées à l'état fondu.

Par exemple, les fibres du milieu de déleucocytation 8 sont choisies parmi les fibres de polyéthylène, polypropylène, polyéthylène téréphtalate, polybutylène téréphtalate et leurs copolymères.

Chaque couche 11 du milieu de déleucocytation présente une perméabilité à l'air inférieure à celle du non-tissé perforé en relief 7, de sorte à créer un gradient de perméabilité à l'air décroissant de l'amont vers l'aval.

Afin de retenir au mieux les leucocytes, chaque couche 11 du milieu de déleucocytation 8 présente une perméabilité comprise dans la plage allant de 90 à 500 L/m²/s.

La masse surfacique de chaque couche 11 du milieu de déleucocytation 8 est comprise dans la plage allant de 20 à 80 g/m², notamment la plage allant de 30 à 60 g/m² pour une épaisseur comprise entre 100 et 400 µm.

Un procédé de fabrication d'une couche de non-tissé perforé en relief pour la filtration du sang ou d'un composant sanguin avec une unité de filtration telle que décrite ci-dessus comprend l'embossage et le perçage d'une couche de non-tissé entre deux rouleaux dont l'un au moins est pourvu d'une pluralité d'aiguilles, de sorte à perforer le non-tissé en créant des bosses 9 autour des perforations qui sont en élévation par rapport à la surface du non-tissé.

Pour faciliter et consolider la création des bosses 9 perforées sur le non-tissé, l'un des rouleaux comprend une pluralité d'aiguilles et l'autre rouleau comprend des cavités dont la géométrie est adaptée à l'emboîtement desdites aiguilles lors de l'embossage. En outre, il est profitable que l'un au moins des rouleaux soit chauffé.

La couche de non-tissé perforé en relief ainsi obtenue par ce procédé de fabrication est utilisée pour éliminer les agrégats du sang ou d'un composant sanguin destiné à être filtre.

En particulier, cette couche de non-tissé perforé en relief est assemblée avec un milieu de déleucocytation pour former un élément poreux, ledit élément poreux étant ensuite disposé dans une enveloppe extérieure d'une unité de filtration.

Un procédé de fabrication d'une unité de filtration 1 selon le premier aspect comprend ainsi :
- la formation d'une couche de non-tissé perforé en relief par embossage et perçage d'une couche de non-tissé entre deux rouleaux dont l'un au moins est pourvu d'une pluralité d'aiguilles, de sorte à perforer le non-tissé en créant des bosses 9 autour des perforations 10 qui sont en élévation par rapport à la surface du non-tissé;
- l'assemblage de ladite couche de non-tissé perforé en relief avec un milieu de déleucocytation de sorte à former un élément poreux, et
- la disposition dudit élément poreux dans une enveloppe extérieure d'une unité de filtration.

L'invention porte aussi sur un procédé de déleucocytation du sang ou d'un composant sanguin comprenant le passage dudit sang ou dudit composant sanguin au travers d'une unité de filtration telle que décrite ci-dessus.

Le procédé de déleucocytation est un procédé extracorporel, réalisé en dehors du corps humain, une fois le sang ou le composant sanguin extrait et isolé du donneur. Le sang ou le composant sanguin à déleucocyter est contenu dans une poche.

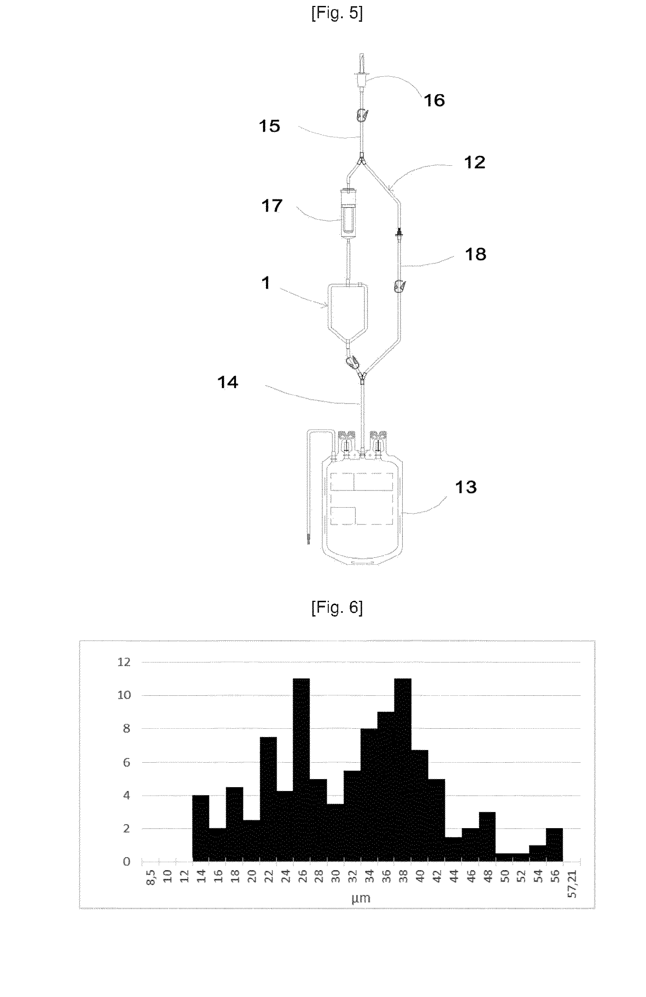

Selon un autre aspect et en relation avec la figure 5, l'invention concerne en outre un système à poches 12 pour la déleucocytation d'un fluide tel que le sang ou un composant sanguin, comprenant une poche 13 de recueil du filtrat, ladite poche 13 étant reliée, par l'intermédiaire d'une tubulure 14 et au niveau d'un orifice d'entrée, à un orifice de sortie d'une unité de filtration 1 selon le premier aspect de l'invention.

Le système 12 comprend en outre des moyens de connexion avec une poche (non représentée) contenant le fluide à filtrer qui sont connectés, par l'intermédiaire d'une tubulure 15, à un orifice d'entrée de l'unité de filtration. Les moyens de connexion sont par exemple un perforateur 16.

En alternative, une poche (non représentée) destinée à contenir le fluide à filtrer est pré-connectée à l'unité de filtration 1 par l'intermédiaire de la tubulure 15.

Ainsi, le fluide, une fois collecté et transféré dans une poche, peut être introduit dans le système à poches 12 pour être filtré au moyen de l'unité de filtration, le filtrat étant ensuite recueilli dans la poche de recueil du filtrat 13.

Une chambre compte-gouttes 17 est connectée au système sur la tubulure 15 reliant l'unité de filtration 1 et les moyens de connexion à une poche contenant le fluide à filtrer.

Une tubulure de dérivation 18 est connectée d'une part à la tubulure 14 reliant l'unité de filtration 1 et la poche 13 de recueil du filtrat, et d'autre part à la tubulure 15 reliant l'unité de filtration et les moyens de connexion à une poche contenant le fluide à filtrer, en amont de la chambre compte-gouttes 17, le cas échéant.

Cette tubulure de dérivation 18 est utilisée pour chasser l'air de la poche 13 de recueil du filtrat et pour purger l'unité de filtration 1.

D'autres systèmes à poches bien connus, comme ceux décrits dans le document EP 1 336 417 peuvent être utilisés dans le cadre de l'invention.

### Exemples

### Exemple 1 : Caractérisation des non-tissés perforés en relief

Un non-tissé de type filé lié a été réalisé

Les caractéristiques physiques d'un non-tissé de type filé lié (SB) et d'un non-tissé de type fibres soufflées à l'état fondu (MB) avant et après perforation sont indiquées ci-dessous :

**[Tableau 1]**

| | Epaisseur (µm) | Perméabilité à l'air (L/m²/s à 196 Pa) |
|---|---|---|
| SB non perforé | 330 | > 800 |
| SB7 (7 perforation/cm²) | 680 | 2350-2370 |
| SB11 (11 perforations/cm²) | 670 | 2450-2490 |
| 2SB11 | 1200 | 1480-1540 |
| 2 couches de PET11 | | |
| MB non perforé | 430 | > 300 |
| MB7 (7 perforations/cm²) | 430 | 1250-1480 |
| 2MB7 | 800 | 590-650 |
| 2 couches de MB7 | | |
| MB11 (11 perforations/cm²) | 540 | 1320-1810 |
| 2MB11 | 990 | 800-920 |
| 2 couches de MB11 | | |

Les perforations en relief ont été caractérisées à l'aide d'un poromètre extrusion liquide de la société PMI.

Les résultats analytiques sont les suivants :

**[Tableau 2]**

| Pores | SB | SB11 | MB11 |
|---|---|---|---|
| Moyenne (µm) | 34 | 161 | 15 |
| Diamètre Minimum (µm) | 8,5 | 8,4 | 3,7 |
| Diamètre Maximum (µm) | 57 | 459 | 208 |
| Mode | 38-40 µm | 440-450 µm | 10-20 µm |

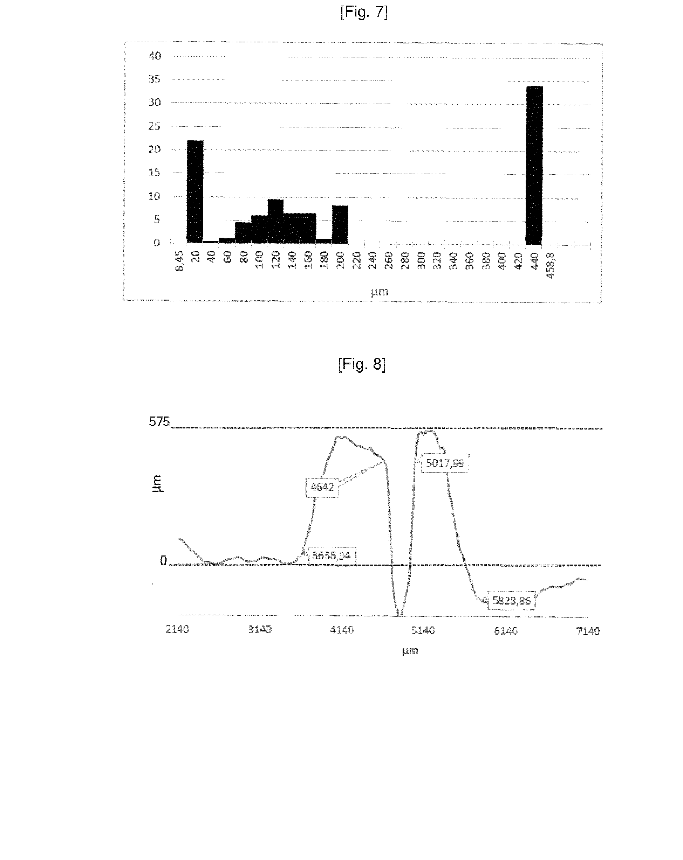

La figure 6 représente la distribution de la taille de pores du non-tissé SB avant perforation et la figure 7, la distribution de la taille des pores du non-tissé SB11.

La figure 8 représente la caractérisation optique du SB11. Selon cette caractérisation optique, la hauteur d'une bosse 9 est d'environ 575 µm, le diamètre de l'ouverture distale de la perforation est d'environ 375 µm et le diamètre de l'ouverture proximale de la perforation d'environ 2200 µm.

### Exemple 2 : filtration d'un concentré de globules rouges conservé 7 jours à froid

Une première série d'essais a été réalisée afin de tester les performances d'une unité de filtration selon l'invention comprenant une ou deux couches de non-tissé perforé en relief.

Le composant sanguin à déleucocyter est un concentré de globules rouges obtenu par centrifugation douce (2000g) d'un prélèvement de sang total (450-480 ml) additionné d'un anticoagulant (CPD). Afin de favoriser la formation d'agrégats, le concentré de globules rouges est conservé sans ajout de solution additive 7 jours à 4°C avant la filtration. La filtration est réalisée à température ambiante, le concentré de globules ayant une température d'environ 12-15°C.

### Unité de filtration de référence 1

Une unité de filtration a été réalisée comprenant, dans un boîtier rigide, un élément poreux constitué d'amont en aval et empilées l'une sur l'autre :
- un voile cardé consolidé par traversée d'air comprenant un mélange de fibres de PET et de fibres Co-PET ayant une perméabilité comprise entre 4000 et 5000 L/m²/s, une masse surfacique comprise entre 50 et 70 g/m², et une taille moyenne de pores d'environ 100 µm ;
- deux couches de non-tissé melt-blown en polypropylène ayant une épaisseur de l'ordre de 285 µm, et une perméabilité à l'air d'environ 800 L/m²/s, en tant que couches de préfiltre ;
- seize couches de non-tissé en polypropylène melt-blown ayant chacune une masse surfacique d'environ 40 g/m² et une perméabilité à l'air d'environ 110 l/m²/s, en tant que milieu de déleucocytation.

### Unité de filtration 1

Dans l'unité de filtration, le voile cardé de l'unité de filtration de référence a été remplacé par une couche de non-tissé perforé en relief SB11, avec les bosses 9 dirigées vers l'aval de l'unité de filtration.

### Unité de filtration 2

Dans l'unité de filtration 2, le voile cardé de l'unité de filtration de référence a été remplacé par deux couches de non-tissé perforé SB11, empilées l'une sur l'autre, avec les bosses 9 dirigées vers l'aval de l'unité de filtration.

### Unité de filtration 3

Dans l'unité de filtration 3, le voile cardé de l'unité de filtration de référence a été remplacé par deux couches de non-tissé perforé SB11 avec les bosses 9 dirigées vers l'amont de l'unité de filtration.

### Unité de filtration 4

Dans l'unité de filtration 4, le voile cardé de l'unité de filtration de référence a été remplacé par une couche de non-tissé perforé SB7. Les bosses 9 sont dirigées vers l'aval de l'unité de filtration.

### Unité de filtration 5

Dans l'unité de filtration 5, le voile cardé de l'unité de filtration de référence a été remplacé par deux couches de non-tissé de type fibres soufflées à l'état fondu MB7, avec les bosses 9 dirigées vers l'aval de l'unité de filtration.

### Unité de filtration 6

Dans l'unité de filtration 6, le voile cardé de l'unité de filtration de référence a été remplacé par une couche de non-tissé MB11 avec les bosses 9 dirigées vers l'aval.

### Unité de filtration 7

Dans l'unité de filtration 7, le voile cardé de l'unité de filtration de référence a été remplacé par deux couches de non-tissé perforé MB11 empilées l'une sur l'autre, avec les bosses 9 dirigées vers l'aval de l'unité de filtration.

Les résultats de déleucocytation sont montrés dans le tableau 3.

**[Tableau 3]**

| | Nombre d'essais n = | Temps de filtration moyen +/écart-type (min) | Nombre de leucocytes (moyen)/poche | Perte (mL) | Taux de déleucocytation moyen (log réduction) |
|---|---|---|---|---|---|
| Unité de référence 1 | 10 | 51 ± 28 | 2,15. 10⁵ | 20 | 4,47 |
| Unité de filtration 1 | 11 | 49 ± 20 | 6,56. 10⁵ | 19,5 | 4,05 |
| Unité de filtration 2 | 10 | 43 ± 17 | 2,2. 10⁵ | 19,2 | 4,25 |
| Unité de filtration 3 | 11 | 53 ± 30 | 4,07. 10⁵ | 19,2 | 4,17 |
| Unité de filtration 4 | 11 | 35 ± 19 | 3,83. 10⁵ | 18,7 | 4,16 |
| Unité de filtration 5 | 3 | 75 ± 33 | 4,53. 10⁴ | 21,4 | 4,96 |
| Unité de filtration 6 | 3 | 201 ± 150 | 1,93. 10⁶ | 26 | 3,63 |
| Unité de filtration 7 | 5 | 104 ± 94 | 6,21. 10⁴ | 23,8 | 4,37 |

On note que les temps de filtration des unités de filtration 5 à 7 comprenant des couches de non-tissé de type melt-blown perforé sont plus longs que ces des autres unités de filtration comprenant des couches de non-tissé de type spun-bond perforé. Il est ainsi avantageux d'utiliser une couche de non-tissé spun-bond perforé pour retenir les agrégats.

### Exemple 3 : filtration d'un concentré de globules rouges non appauvri en leucocytes et en plaquettes

Une autre série d'essais a été réalisée afin de tester les performances d'une unité de filtration en système clos selon l'invention comprenant une ou deux couches de non-tissé perforé en relief.

Le composant sanguin à déleucocyter est un concentré de globules rouges non appauvri en leucocytes et en plaquettes obtenu par centrifugation dure d'un prélèvement de sang total (450-480 ml) additionné d'un anticoagulant (CPD) et conservé à 4°C pendant 3 jours. Le concentré de globules rouges est séparé du plasma et le mélange concentré de globules rouge / couche leuco-plaquettaire est filtré à température ambiante.

### Unité de filtration de référence 2

Une unité de filtration a été réalisée comprenant, dans une enveloppe souple, un élément poreux constitué d'amont en aval et empilées l'une sur l'autre :
- deux couches de non-tissé filé lié en polyester ayant une épaisseur d'environ 330 µm, et une perméabilité à l'air supérieure à 800 L/m²/s.
- 26 couches de non-tissé de fibres soufflées à l'état fondu en polypropylène formant un gradient décroissant de perméabilité à l'air allant d'environ 400 à environ 70 L/m²/s
- une couche de non-tissé filé lié tissé ayant une épaisseur d'environ 330 µm et une perméabilité à l'air supérieure à 800 L/m²/s.

### Unité de filtration 8

Dans l'unité de filtration 8, les deux premières couches de non-tissé filé lié de l'unité de filtration de référence 2 ont été remplacées par deux couches SB11 avec les bosses 9 dirigées vers l'aval de l'unité de filtration.

Les résultats de déleucocytation sont montrés dans le Tableau 4.

**[Tableau 4]**

| | Nombre d'essais n = | Temps de filtration moyen +/écart-type (min) | Perte (mL) | Blocage (%) | Taux de récupération des globules rouges (%) |
|---|---|---|---|---|---|
| Unité de filtration de référence 2 | 10 | - | - | 10 | 82 |
| Unité de filtration 8 | 7 | 126+/- 41 | 29,8 | 0 | 90 |

### Exemple 4 : filtration d'un concentré de globules rouges à température ambiante

Dans cet essai, le composant sanguin à déleucocyter est un concentré de globules rouges obtenu par centrifugation douce (2 600g) d'un prélèvement de sang total (450-480 ml) additionné d'un anticoagulant (CPD). Le concentré de globules rouges est séparé du plasma riche en plaquettes, mélangé avec une solution additive de type SAGM et filtré à température ambiante. La filtration est réalisée dans les 8 heures suivant le prélèvement de sang avec l'unité de filtration 8.

Les résultats de déleucocytation sont montrés dans le Tableau 5.

**[Tableau 5]**

| | Nombre d'essais n = | Temps de filtration moyen (min) | Perte (mL) | Blocage (%) | Taux de récupération des globules rouges (%) | Nombre de globules blancs |
|---|---|---|---|---|---|---|
| Unité de filtration 8 | 1 | 49 | 25 | 0 | 93 | 1,61.10⁵ |

## Revendications

1. Unité de filtration (1) destinée à permettre la déleucocytation du sang ou d'un composant sanguin, comprenant une enveloppe extérieure (2) munie d'au moins un orifice d'entrée (3) et d'au moins un orifice de sortie (4), l'enveloppe renfermant un élément poreux interposé entre lesdits orifices, ledit élément poreux comprenant au moins un milieu de déleucocytation (8) par adsorption et/ou par filtration des leucocytes, **caractérisée en ce que** ledit élément poreux comprend en outre au moins une couche de non-tissé (7) pourvu d'une pluralité de bosses (9), chacune desdites bosses étant traversées dans la hauteur par une perforation (10), de sorte à former une couche de non-tissé perforé en relief.

2. Unité de filtration selon la revendication 1, **caractérisée en ce que** les bosses (9) sont de forme tronconique.

3. Unité de filtration selon l'une des revendications 1 ou 2, **caractérisée en ce que** les bosses (9) forment sur le non-tissé un motif distinct et répété.

4. Unité de filtration selon l'une quelconque des revendications 1 à 3, **caractérisée en ce que** la couche de non-tissé perforé en relief est disposée en amont du milieu de déleucocytation (8).

5. Unité de filtration selon l'une quelconque des revendications 1 à 4, **caractérisée en ce que** le non-tissé perforé en relief possède une épaisseur comprise dans la plage allant de 500 à 1 300 µm.

6. Unité de filtration selon l'une quelconque des revendications 1 à 5, **caractérisée en ce que** le non-tissé perforé en relief présente une masse surfacique comprise dans la plage allant de 40 à 90 g/m².

7. Unité de filtration selon l'une quelconque des revendications 1 à 6, **caractérisée en ce que** le non-tissé perforé en relief présente une perméabilité à l'air comprise dans la plage allant de de 800 à 3 000 L/m²/s.

8. Unité de filtration selon l'une quelconque des revendications 1 à 7, **caractérisée en ce que** les bosses (9) s'étendent à partir de la surface du non-tissé sur une hauteur comprise entre 200 µm et 1 500 µm.

9. Unité de filtration selon l'une quelconque des revendications 1 à 8, **caractérisée en ce que** le non-tissé perforé en relief comprend un taux de perforation compris dans la plage allant de 5 à 20 perforations par cm².

10. Unité de filtration selon l'une quelconque des revendications 1 à 9, **caractérisée en ce que** le non-tissé perforé en relief est un non-tissé filé lié.

11. Unité de filtration selon l'une quelconque des revendications 1 à 10, **caractérisée en ce que** le milieu de déleucocytation (8) comprend une ou plusieurs couches (11) d'un matériau non-tissé de fibres soufflées à l'état fondu.

12. Unité de filtration selon l'une quelconque des revendications 1 à 11, **caractérisée en ce que** le milieu de déleucocytation (8) comprend une ou plusieurs couches (11) d'un matériau non-tissé, chaque couche (11) du milieu de déleucocytation présentant une perméabilité comprise dans la plage allant de 90 à 500 L/m²/s.

13. Unité de filtration selon l'une quelconque des revendications 1 à 12, caractérisée le milieu de déleucocytation (8) comprend une ou plusieurs couches (11) d'un matériau non-tissé, chaque couche (11) du milieu de déleucocytation présentant une perméabilité à l'air inférieure à celle de la couche de non-tissé perforé en relief.

14. Procédé de fabrication d'une unité de filtration (1) selon l'une quelconque des revendications 1 à 13, **caractérisé en ce qu'**il comprend :
- la formation d'une couche de non-tissé perforé en relief par embossage et perçage d'une couche de non-tissé entre deux rouleaux dont l'un au moins est pourvu d'une pluralité d'aiguilles, de sorte à perforer le non-tissé en créant des bosses (9) autour des perforations (10) qui sont en élévation par rapport à la surface du non-tissé;
- l'assemblage de ladite couche de non-tissé perforé en relief avec un milieu de déleucocytation de sorte à former un élément poreux, et
- la disposition dudit élément poreux dans une enveloppe extérieure d'une unité de filtration.

15. Procédé de fabrication selon la revendication 14, **caractérisé en ce que** l'un des rouleaux comprend une pluralité d'aiguilles et l'autre rouleau comprend des cavités dont la géométrie est adaptée à l'emboîtement des aiguilles lors de l'embossage.

16. Procédé extracorporel de déleucocytation du sang ou d'un composant sanguin comprenant le passage dudit sang ou dudit composant sanguin au travers d'une unité de filtration (1) selon l'une quelconque des revendications 1 à 13, ledit sang ou ledit composant sanguin étant contenu dans une poche.

17. Système à poches (12) pour la déleucocytation du sang ou d'un composant sanguin, **caractérisé en ce qu'**il comprend une poche (13) de recueil du filtrat, ladite poche (13) étant reliée, par l'intermédiaire d'une tubulure (14) et au niveau d'un orifice d'entrée, à un orifice de sortie d'une unité de filtration (1) selon l'une des revendications 1 à 13.

## Patentansprüche

1. Filtrationseinheit (1), die dazu bestimmt ist, die Leukozytapharese des Blutes oder einer Blutkomponente zu ermöglichen, umfassend eine äußere Hülle (2), die mit mindestens einer Einlassöffnung (3) und mindestens einer Auslassöffnung (4) versehen ist, wobei die Hülle ein zwischen den Öffnungen eingefügtes poröses Element umschließt, wobei das poröse Element mindestens ein Mittel zur Leukozytapharese (8) durch Adsorption und/oder durch Filtration von Leukozyten umfasst, **dadurch gekennzeichnet, dass** das poröse Element ferner mindestens eine Schicht von Vliesstoff (7) umfasst, die mit einer Vielzahl von Ausprägungen (9) versehen ist, wobei jede der Ausprägungen in der Höhe von einer Perforation (10) durchquert wird, um eine Schicht aus relief-perforiertem Vliesstoff zu bilden.

2. Filtrationseinheit nach Anspruch 1, **dadurch gekennzeichnet, dass** die Ausprägungen (9) kegelstumpfförmig sind.

3. Filtrationseinheit nach einem der Ansprüche 1 oder 2, **dadurch gekennzeichnet, dass** die Ausprägungen (9) auf dem Vliesstoff ein deutliches, sich wiederholendes Muster bilden.

4. Filtrationseinheit nach einem der Ansprüche 1 bis 3, **dadurch gekennzeichnet, dass** die Schicht aus relief-perforiertem Vliesstoff oberhalb des Mittels zur Leukozytapharese (8) angeordnet ist.

5. Filtrationseinheit nach einem der Ansprüche 1 bis 4, **dadurch gekennzeichnet, dass** der relief-perforierte Vliesstoff eine Dicke im Bereich von 500 bis 1.300 µm besitzt.

6. Filtrationseinheit nach einem der Ansprüche 1 bis 5, **dadurch gekennzeichnet, dass** der relief-perforierte Vliesstoff ein Flächengewicht im Bereich von 40 bis 90 g/m² aufweist.

7. Filtrationseinheit nach einem der Ansprüche 1 bis 6, **dadurch gekennzeichnet, dass** der relief-perforierte Vliesstoff eine Luftdurchlässigkeit im Bereich von 800 bis 3.000 L/m²/s aufweist.

8. Filtrationseinheit nach einem der Ansprüche 1 bis 7, **dadurch gekennzeichnet, dass** sich die Ausprägungen (9) von der Oberfläche des Vliesstoffs über eine Höhe im Bereich von 200 µm und 1.500 µm erstrecken.

9. Filtrationseinheit nach einem der Ansprüche 1 bis 8, **dadurch gekennzeichnet, dass** der relief-perforierte Vliesstoff eine Perforationsrate im Bereich von 5 bis 20 Perforationen pro cm² umfasst.

10. Filtrationseinheit nach einem der Ansprüche 1 bis 9, **dadurch gekennzeichnet, dass** der relief-perforierte Vliesstoff ein Spinnvliesstoff ist.

11. Filtrationseinheit nach einem der Ansprüche 1 bis 10, **dadurch gekennzeichnet, dass** das Mittel zur Leukozytapharese (8) eine oder mehrere Schichten (11) von einem Vliesmaterial aus schmelzgeblasenen Fasern umfasst.

12. Filtrationseinheit nach einem der Ansprüche 1 bis 11, **dadurch gekennzeichnet, dass** das Mittel zur Leukozytapharese (8) eine oder mehrere Schichten (11) aus einem Vliesstoffmaterial umfasst, wobei jede Schicht (11) des Mittels zur Leukozytapharese eine Permeabilität im Bereich von 90 bis 500 L/m²/s aufweist.

13. Filtrationseinheit nach einem der Ansprüche 1 bis 12, **dadurch gekennzeichnet, dass** das Mittel zur Leukozytapharese (8) eine oder mehrere Schichten (11) von einem Vliesstoffmaterial umfasst, wobei jede Schicht (11) des Mittels zur Leukozytapharese eine geringere Luftdurchlässigkeit zu der der Schicht aus relief-perforiertem Vliesstoff aufweist.

14. Verfahren zur Herstellung einer Filtrationseinheit (1) nach einem der Ansprüche 1 bis 13, **dadurch gekennzeichnet, dass** es umfasst :
- die Bildung einer Schicht aus relief-perforiertem Vliesstoff durch Prägen und Perforieren einer Schicht aus Vliesstoff zwischen zwei Walzen, von denen mindestens eine mit einer Vielzahl von Nadeln versehen ist, um den Vliesstoff zu perforieren, wobei um die Perforationen (10) herum Ausprägungen (9) erzeugt werden, die in Bezug auf die Oberfläche des Vliesstoffs erhöht sind;
- das Zusammenfügen der Schicht des relief-perforierten Vliesstoffs mit einem Mittel zur Leukozytapharese, um ein poröses Element zu bilden, und
- die Anordnung des porösen Elements in einer Außenhülle einer Filtrationseinheit.

15. Herstellungsverfahren nach Anspruch 14, **dadurch gekennzeichnet, dass** eine der Walzen eine Vielzahl von Nadeln umfasst und die andere Walze Hohlräume umfasst, deren Geometrie an das Ineinandergreifen der Nadeln beim Prägen angepasst ist.

16. Extrakorporales Verfahren zur Leukozytapharese des Blutes oder einer Blutkomponente, umfassend die Durchleitung des Blutes oder der Blutkomponente durch eine Filtrationseinheit (1) nach einem der Ansprüche 1 bis 13, wobei das Blut oder die Blutkomponente in einem Beutel enthalten ist.

17. System mit Beuteln (12) zur Leukozytapharese des Blutes oder einer Blutkomponente, **dadurch gekennzeichnet, dass** es einen Beutel (13) zum Sammeln des Filtrats umfasst, wobei der Beutel (13) über einen Zwischenschlauch (14) und auf der Ebene einer Eingangsöffnung mit einer Ausgangsöffnung einer Filtrationseinheit (1) nach einem der Ansprüche 1 bis 13 verbunden ist.

## Claims

1. Filtration unit (1) intended to allow the leucodepletion of the blood or of a blood product, comprising an external pouch (2) provided with at least one inlet orifice (3) and with at least one outlet orifice (4), the pouch containing a porous element interposed between said orifices, said porous element comprising at least one leucodepletion medium (8) that works by adsorbing and/or by filtering out the leucocytes, **characterised in that** said porous element further comprises at least one layer of nonwoven (7) provided with a plurality of bumps (9), each of said bumps having a perforation (10) passing through it in the heightwise direction to form a relief-perforated nonwoven layer.

2. Filtration unit according to claim 1, **characterised in that** the bumps (9) are frustoconical in shape.

3. Filtration unit according to one of claims 1 or 2, **characterised in that** the bumps (9) form a distinct and repeated pattern on the nonwoven.

4. Filtration unit according to any one of claims 1 to 3, **characterised in that** the layer of relief-perforated nonwoven is disposed upstream of the leucodepletion medium (8) .

5. Filtration unit according to any one of claims 1 to 4, **characterised in that** the relief-perforated nonwoven has a thickness in the range from 500 to 1300 µm.

6. Filtration unit according to any one of claims 1 to 5, **characterised in that** the relief-perforated nonwoven has a mass per unit area in the range from 40 to 90 g/m².

7. Filtration unit according to any one of claims 1 to 6, **characterised in that** the relief-perforated nonwoven has an air permeability in the range from 800 to 3000 L/m²/s.

8. Filtration unit according to any one of claims 1 to 7, **characterised in that** the bumps (9) extend from the surface of the nonwoven to a height between 200 µm and 1500 µm.

9. Filtration unit according to any one of claims 1 to 8, **characterised in that** the relief-perforated nonwoven has a perforation rate in the range from 5 to 20 perforations per cm².

10. Filtration unit according to any one of claims 1 to 9, **characterised in that** the relief-perforated nonwoven is a spun-bond nonwoven.

11. Filtration unit according to any one of claims 1 to 10, **characterised in that** the leucodepletion medium (8) comprises one or more layers (11) of a melt-blown fibre nonwoven material.

12. Filtration unit according to any one of claims 1 to 11, **characterised in that** the leucodepletion medium (8) comprises one or more layers (11) of a nonwoven material, each layer (11) of the leucodepletion medium having a permeability in the range from 90 to 500 L/m²/s.

13. Filtration unit according to any one of claims 1 to 12, **characterised in that** the leucodepletion medium (8) comprises one or more layers (11) of a nonwoven material, each layer (11) of the leucodepletion medium having an air permeability less than that of the layer of relief-perforated nonwoven.

14. Method for manufacturing a filtration unit (1) according to any one of claims 1 to 13, **characterised in that** it comprises:
- forming a layer of relief-perforated nonwoven by embossing and piercing a layer of nonwoven between two rollers, at least one of which is provided with a plurality of needles, so as to perforate the nonwoven while creating bumps (9) around the perforations (10) which are elevated relative to the surface of the nonwoven;
- assembling said layer of relief-perforated nonwoven with a leucodepletion medium so as to form a porous element, and
- disposing said porous element in an external pouch of a filtration unit.

15. Manufacturing method according to claim 14, **characterised in that** one of the rollers comprises a plurality of needles and the other roller comprises cavities, the geometry of which is suitable for the interlocking of the needles during the embossing.

16. Extracorporeal method for leucodepletion of the blood or a blood product comprising the passing of said blood or said blood product through a filtration unit (1) according to any one of claims 1 to 13, said blood or said blood product being contained in a bag.

17. Bag system (12) for leucodepletion of the blood or a blood product, **characterised in that** it comprises a bag (13) for collecting the filtrate, said bag (13) being connected, by means of a tube (14) and at an inlet orifice, to an outlet orifice of a filtration unit (1) according to one of claims 1 to 13.
